(19) Europäisches Patentamt
European Patent Office
Office européen des brevets

(11) **EP 0 254 593 B1**

(12) **EUROPEAN PATENT SPECIFICATION**

(45) Date of publication and mention
of the grant of the patent:
**29.04.1998 Bulletin 1998/18**

(51) Int Cl.6: **C12P 21/02**, C12P 21/00,
C12N 15/00, C07K 1/00,
A61K 38/21

(21) Application number: **87306585.8**

(22) Date of filing: **24.07.1987**

(54) **Preparation and uses of interferon-gamma**

Herstellung und Verwendungen von gamma-Interferon

Préparation et applications d'interféron gamma

(84) Designated Contracting States:
**BE CH DE ES IT LI NL SE**

(30) Priority: **25.07.1986 JP 176266/86**
**25.05.1987 JP 125777/87**

(43) Date of publication of application:
**27.01.1988 Bulletin 1988/04**

(73) Proprietor: **KABUSHIKI KAISHA HAYASHIBARA**
**SEIBUTSU KAGAKU KENKYUJO**
**Okayama-shi Okayama (JP)**

(72) Inventors:
• **Kurimoio, Masashi**
**Okayama-shi Okayama (JP)**
• **Mitsuhashi, Masakazu**
**Okayama-shi Okayama (JP)**

(74) Representative: **Pendlebury, Anthony et al**
**PAGE, WHITE & FARRER**
**54 Doughty Street**
**London WC1N 2LS (GB)**

(56) References cited:
EP-A- 0 122 628        EP-A- 0 177 910
GB-A- 2 040 292

• CHEMICAL ABSTRACTS, vol. 94, no. 23, 8th
June 1981, page 486, abstract no. 190203e,
Columbus, Ohio, US; M.
STEWART-WIRANOWSKA et al.: "Determination
of human leukocyte populations involved in
priduction of interferons alpha and gamma", & j.
INTERFERON RES. 1981, 1(2), 233-44
• BIOCHEMISTRY, vol. 25, no. 11, 6th June 1986,
pages 3424-3429, American Chemical Society,
Easton, PA, US; K. MATSUSHIMA et al.:
"Purification and biochemical characteristics of
two distinct human interleukins 1 from the
myelomonocytic THP-1 cell line"
• Methods in Enzymology, vol. 119, pp. 93-95, 1986
• J. Biol. Chem., vol. 259, pp. 6790-6797, 1994
• WHO Expert Committee on Biol.
Standardization, pp. 24-25,1985
• Expert Translation of " Rinsho-Kensa ", vol. 28,
pp. 1729-1731, 1984
• Expert Translation of " Protein, Nucleic acid and
Enzyme ", vol. 20, pp. 616-643, 1975
• ATCC Catalogue, 1983, p. 50
• PNAS USA, vol. 81, pp. 5086-5090, 1984

**Description**

Background of the Invention

1. Field of the invention

The present invention relates to preparation and uses of interferon-gamma.

More particularly, the present invention relates to a process for preparing interferon-gamma, characterized by allowing an established human myelomonocyte of producing interferon-gamma to produce interferon-gamma, and recovering the accumulation; a process to prepare monoclonal anti-interferon-gamma antibody using the same; and a method to purify interferon-gamma using the monoclonal antibody, as well as to a prophylactic and therapeutic agent for interferon-gamma susceptive disease containing the interferon-gamma as an effective ingredient.

2. Description of the prior art

As described in Shigeyasu Kobayashi, "Interferon", published by Kodansha Co., Ltd., Tokyo, Japan (1975), D.A. J. Tyrrell, "Interferon and its Clinical Potential", published by William Heinemann Medical Books Ltd., London (1976), and Protein, Nucleic Acid and Enzyme, Vol.21, No.4, pp.245-333 (1976), interferon is a name to designate glycoproteins that are extracellularly inducible in viable cell by subjecting it to the action of an interferon inducer, for example, virus, bacetrium, protozoon, rickettsia, nucleic acid, endotoxin and polysaccharide, as well as having an activity of nonspecifically inhibiting viral growth.

This activity has rendered interferons since the discovery a potential prophylactic and therapeutic agent for viral diseases. Recent studies revealed that interferons exert an antioncotic activity on viral tumors, as well as on nonviral tumors. Because of the activity, the development of pharmaceuticals using interferons is in great expectation.

Interferons include interferon-alpha (or leukocyte interferon), interferon-beta (or fibroblast interferon), and interferon-gamma (or immune interferon). Preparation of interferon-alpha and interferon-beta has been established by using leukocyte and fibroblast cell. Recently, pharmaceuticals incorporated with these interferons have been commercialized.

Respective interferon will hereinafter be abbreviated as "IFN-alpha", "IFN-beta" and "IFN-gamma" occasionally with the prefix "Hu" representing human origin.

Although various methods have been proposed for preparing HuIFN-gamma, no method has been practiced on industrial scale.

The methods using leukocyte or T lymphocyte derived from human peripheral blood, as proposed, for example, in Japanese Patent Laid-Open Nos.58,891/82, 82,092/84, 70,099/85, 87,300/85, 139,700/85 and 149,600/85, International Patent Publication in Japanese Nos.500,961/82 and 502,032/83, are practically unfavorable because an ample supply of the material cell is very difficult and the HuIFN producibility of these cells is insufficient.

Japanese Patent Laid-Open No.98,118/80 proposes the method wherein a human cell, obtained by implanting an established human cell in a non-human warm-blooded animal or placing the cell in a diffusion chamber provided inside or outside the body of a non-human warm-blooded animal, and allowing the cell to proliferate while allowing the cell to receive the nutrient body fluid from the animal, is used in preparing HuIFN-gamma. This method is characterized by an ample supply of the material human cells.

We found that the HuIFN-gamma productivity of the method varies with the type of the human cell used. Thus, the method has room for improvement in preparing consistently high-titered HuIFN-gamma to be practiceable on industrial scale.

It is known that HuIFN-gamma is much more stronger in cytostatic and antioncotic activities than HuIFN-alpha and HuIFN-beta. Also is known that combination with HuIFN-alpha and/or HuIFN-beta augments the antiviral, cytostatic and antioncotic activities of HuIFN-gamma. For these reasons, development of an industrial-scale preparation of HuIFN-gamma has been in great demand.

Summary of the Invention

In view of the foregoing, we screened various established human cells, specifically, established human lymphoblastoid cells, for their HuIFN-gamma producibility that may facilitate industrial-scale preparation of HuIFN-gamma, as well as investigating the potentiality as prophylactic and therapeutic agent for HuIFN-gamma susceptive diseases.

As the result, we unexpectedly found that established human myelomonocytes exert a higher HuIFN-gamma producibility than other lymphoblastoid cells and are suitable for HuIFN-gamma producer cell, as well as that the obtained HuIFN-gamma is superiorly efficacious when used in prophylactic and therapeutic agent for HuIFN-gamma susceptive diseases.

The present invention provides a process for preparing interferon-gamma, comprising:

allowing an established human myelomonocyte capable of producing interferon-gamma to produce interferon-gamma; and
and recovering the accumulation.

The present invention also provides a prophylactic and therapeutic agent for interferon-gamma susceptive diseases, said agent containing as an effective ingredient 1-10,000,000 units of the interferon-gamma per gram of the agent, which interferon-gamma is obtainable by a process according to any one of claims 1 to 4, wherein the interferongamma activity is determined by the plaque reduction method using FL cells derived from human amnion, and a unit of the interferon-gamma is defined as the amount that gives a plaque reduction rate (%) of about 50% when calculated by the equation below:

$$\text{Plaque reduction rate (\%)} = \frac{A - B}{A} \times 100$$

wherein "A" designates the plaque number with no addition of the interferon-gamma; and "B", that with addition of the interferon-gamma.

Brief Explanation of the Drawings

FIG.1 is the phase-contrast microscopic view of HBL-38 cell.
FIG.2 shows the results of karyotypic analysis on HBL-38 cell.

Detailed Description of the Invention

The wording "established human myelomonocyte" means those which are not grouped into T and B cells and identifiable by checking the presence of myelomonocytic antigen by antigen-antibody reaction; for example, those as described in Iwanami Koza Men-eki Kagaku (The Iwanami Immunology Series), Vol.3, "Immunity responsive cells", edited by Tadamitsu Kishimoto and Takeshi Watanabe, pp.181-204, published by Iwanamishoten Publisher, Tokyo, Japan (1986), and "Mammalian Cell Culture Technology", written by Mikio Shikita and Isao Yamane, pp.141-162, published by Soft Science Publications, Tokyo, Japan (1985).

Examples of such myelomonocyte include HBL-38 cell established by us; HL-60 cell, KG-1 cell, ML-1 cell, ML-2 cell, ML-3 cell, THP-1 cell and U-937 cell as described in the above references; and CTV-1 cell as reported in Japanese Journal of Cancer Research (Gann), Vol. 75, pp.660-664 (1984). Particularly, HBL-38 cell is most favorable in practicing the present invention because it exerts a higher HuIFN-gamma producibility. In order to augment the proliferation rate and HuIFN-gamma producibility, the gene coding HuIFN-gamma production can be introduced into a readily subculturable established human cell by cell fusion using, for example, polyethylene glycol or Sendai virus, or by gene recombinant technique using DNA ligase, restriction enzyme (nuclease) and DNA polymerase.

Proliferation method for human myelomonocyte can be suitably selected. Examples of such method include the tissue culture method wherein human myelomonocyte is inoculated on a nutrient culture medium and cultured in vitro; and the method wherein human myelomonocyte is implanted in a non-human warm-blooded animal or a diffusion chamber placed inside or outside the body of a non-human warm-blooded animal, and then allowed to proliferation while receiving the nutrient body fluid from the animal.

First, the in vitro proliferation will be explained

In the in vitro proliferation, any nutrient culture medium can be used as long as human myelomonocyte proliferates therein, for example, RPMI 1640 medium and Eagle's minimal essential medium. These culture media may be modified by supplementing it with vitamin, mineral, carbohydrate, amino acid and/or mammalian serum.

The culture may be a monolayer or suspension culture. The temperature is about 20-40°C, desirably, about 35-38°C, and the inoculum should have a cell number per ml culture medium that attains a maximum proliferation over a period of about one week perferably, about $10^4$-$10^7$ cells/ml culture medium.

The culture medium containing human myelomonocyte is cultured under these conditions for about 4-10 days, and, during the culture, the culture medium may be successively refreshed to supplement sufficient amounts of nutrients, as well as to wash and/or dilute the metabolites released in the culture medium.

The in vivo proliferation is now explained.

The human myelomonocytes can be easily proliferated with the in vivo proliferation by implanting it in a non-human warm-blooded animal or placing it in a diffusion chamber in which the cell be supplied with the nutrient body fluid of the animal, and feeding the animal in usual manner. The in vivo proliferation attains a larger amount of HuIFN-gamma with no or much less nutrient culture medium containing expensive serum than in vivo culture.

The in vivo proliferation has the additional advantages that it reduces cares during cell proliferation; that it stabilizes cell proliferation; and that it augments HuIFN-gamma producibility per cell, particularly, two- to ten-folds or more.

The non-human warm-blooded animals usable in the in vivo proliferation are those wherein human myelomonocytes proliferate, for example, fowls such as chicken and pigeon, and mammals such as dog, cat, monkey, goat, pig, cow, horse, rabbit, guinea pig, rat, hamster, mouse and nude mouse.

Since implantation of human myelomonocyte may elicit an undesirable immunoreaction in the animal, the use of an animal in the youngest possible stage, for example, egg, embryo or fetus, or newborn or infant animal, is desirable in order to reduce the immunoreaction to the lowest possible level.

For the same purposes, the animal may be irradiated with x-ray or gamma-ray, about 200-600 rem, or injected with an antiserum or an immunosuppressant prior to implantation.

When nude mouse is used, human myelomonocyte can be implanted without pretreatment and proliferation readily with less fear of causing undesirable immunoreaction because nude mouse elicits less immunoreaction even in adulthood.

One can stabilize cell proliferation and/or augment HuIFN-gamma production by successive implantation using the same or different non-human warm-blooded animals. These can be attained by, for example, first implanting and proliferating human myelomonocyte in hamster, then successively implanting the proliferated cell in nude mouse. Such implantation can be carried out with non-human warm-blooded animals of the same class or order, as well as those of the same species or genus.

Human myelomonocyte can be implanted in any site of the animal as long as the cell proliferate in the site; for example, in the allantoic cavity, intravenously, intraperitoneally or subcutaneously.

Alternatively, human myelomonocyte can be proliferated by placing it in conventional diffusion chamber of various shapes and sizes, equipped with an appropriate means which excludes the animal cell but supplies to HBL-38 the nutrient body fluid from a non-human warm-blooded animal, for example, membrane filter, ultrafilter or hollow fiber, pore size of about $10^{-7}$-$10^{-5}$ m; embedding, for example, intraperitoneally, the chamber in a non-human warm-blooded animal; and allowing the cell to proliferate in the chamber while allowing the cell to receive the nutrient body fluid from the animal.

The diffusion chamber can be arranged and placed, for example, on the animal, in such manner that the nutrient fluid in the diffusion chamber can freely circulate therethrough. The diffusion chamber can be arranged in such manner that the culture can be observed during the cell proliferation through the chamber wall, and/or that a diffusion chamber can be replaced at intervals with a fresh one to continue the cell proliferation over the life span of the animal without sacrifice, as well as to augment much more the cell production per animal.

Since in the method using diffusion chamber human myelomonocyte never contacts with the animal cell and elicits much less undesirable immunoreaction, any non-human warm-blooded animal can be freely used without pretreatment to reduce immunoreaction and the proliferated cell can be easily recovered.

The animal is fed in usual manner, and no special care is required even after implantation. The period for maximum cell proliferation is usually from 1-10 weeks. The number of the obtained myelomonocyte is about $10^7$-$10^{12}$ cells per animal or more. More particularly, according to the invention, the implanted myelomonocyte increases about $10^2$-$10^7$-fold or more, which is about 10-$10^6$-folds or higher than that attained by inoculating and proliferation myelomonocyte on in vitro nutrient culture medium,. This is very favorable in preparing HuIFN-gamma.

Any induction method can be employed in the invention as long as it induces HuIFN-gamma production in the human myelomonocytes obtained in this way. The human myelomonocyte can be exposed to the action of IFN-gamma inducer in the animal used for proliferation. For example, a human myelomonocyte proliferated in ascite in suspension, or a tumor, formed, for example, subcutaneously, is exposed directly to IFN-gamma inducer, and the resultant HuIFN-gamms is recovered from the ascite, serum and/or tumor, followed by purification.

The proliferated human myelomonocyte can be recovered from the animal, and then exposed in vitro to IFN-gamma inducer. For example, a human myelomonocyte obtained by recovery from ascite, or extraction and disaggregation of the tumor mass formed, for example, subcutaneously, is suspended in a nutrient culture medium kept at about 20-40°C to give a cell density of about $10^5$-$10^8$ cells/ml, and exposed to IFN-gamma inducer, followed by recovery and purification of the resultant HuIFN-gamma.

When diffusion chamber is used, human myelomonocyte can be exposed to IFN-gamma inducer in the diffusion chamber or after recovery therefrom.

The priming method using HuIFN and/or the super-induction method using antimetabolite can be employed to further augment the production of HuIFN-gamma.

Production of HuIFN-gamma per animal may be still further augmented by employing one or more of the following methods:

(1) a method wherein human myelomonocyte is exposed to IFN-gamma inducer in the animal, recovered from certain site of the animal or its whole body, and exposed in vitro to IFN-gamma inducer,
(2) a method wherein human myelomonocyte is repeatedly exposed to IFN-gamma inducer, and

(3) a method wherein the diffusion chamber embedded in or connected to the animal is replaced at intervals with a fresh chamber.

The IFN-gamma inducers usable in the invention are usually mitogens, for example, phytohemagglutinin, concanavalin A, pokeweed mitogen, lipopolysaccharide, endotoxin, polysaccharide and bacterium.

Antigens act on sensitized cell as IFN-gamma inducer. The IFN-gamma inducers are usually used at a concentration of from about 0.001 μg/ml to 10 mg/ml. Combination with IFN-alpha inducer, for example, virus, nucleic acid and polynucleotide, may lead to an augmented production of HuIFN-gamma and/or induction of a simultaneous production of HuIFN-alpha.

The obtained HuIFN-gamma can be recovered with one or more conventional purification and separation methods, for example, salting-out, dialysis, filtration, centrifugation, concentration and lyophilization. When further purification is required, one or more other conventional procedures, for example, adsorption and desorption with ion exchange, gel filtration, isoelectric point fractionation, electrophoresis, ion exchange chromatography, high-performance liquid chromatography, column chromatography and affinity chromatography, are used in combination. Chromatography using monoclonal antibody leads to an HuIFN-gamma of the highest purity.

The HuIFN-gamma thus obtained can be advantageously used in preventing and treating HuIFN-gamma susceptive diseases.

The wording "HuIFN-gamma susceptive diseases" means those which can be prevented or treated with HuIFN-gamma, for example, viral diseases such as epidemic conjunctivitis, herpetic keratitis, influenza, rubella, serum hepatitis, and acquired immune deficiency syndrome (AIDS); and nonviral diseases including malignant tumors such as colon carcinoma, lung carcinoma, liver carcinoma and osteosarcoma, and immunopathies including atopic allergy, myoasthenia, collagenosis, pernicious anemia, articular rheumatism, and ststemic lupus erythematosus.

The agent according to the invention can be prepared into an appropriate form to meet the final uses, for example, into nebula, collyrium, collutory, a liquid medicine such as injection, a paste medicine such as ointment, and solid medicines such as powder, granule and tablet.

The HuIFN-gamma content is usually in the range of 1-10,000,000 units/g. The efficacy can be augmented by combining one or more lymphokines such as HuIFN-alpha, HuIFN-beta, tumor necrosis factor (TNF), lymphotoxin, interleukin 2, and B-cell differentiating factor, or natural or synthetic chemotherapeutic(s).

The HuIFN-gamma can be used in combination with adjuvant, filler and/or stabilizer. The agent thus obtained is suitable, for example, for antiviral agent, antioncotic, enhancer for antioncotic chemotherapeutics, depressant for metastasis of tumors, suppressant for palindromia, and immunoregulator.

The activity of HuIFN was determined with the plaque reduction method using FL cell derived from human amnion as described in Protein, Nucleic Acid and Enzyme, Vol. 20, No.6, pp.616-643 (1975).

The activity of HuIFN-gamma was determined after neutralization of HuIFN-alpha and HuIFN-beta respectively with anti-HuIFN-alpha- and anti-HuIFN-gamma-antibodies.

The hemagglutination titer was determined in accordance with the method as described in J.E Salk, The Journal of Immunology, Vol.49, pp.87-98 (1944).

HBL-38 cell established by us will hereinafter be described.

After culturing on in vitro nutrient culture medium, a leukocyte from an acute myeloleukemia patient (55-year old) began proliferation on the 21st day. We repeatedly subcultured the leukocyte and eventually succeeded to stably proliferate one of the subcultures. We name the subculture as "HBL-38".

(1) Proliferation

Doubling time on RPMI 1640 medium supplemented with 10 v/v % fetal calf serum was about 30 hours.

(2) Morphology

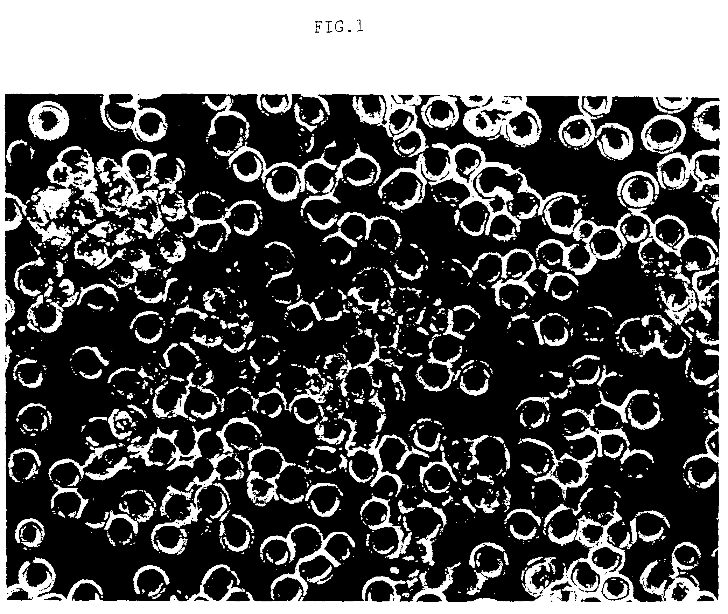

HBL-38 cell tended to attach on the inside bottom of flask during proliferation, but the attachment was loose and the cell was easily detachable. Although cell clumps were formed during proliferation, they were not rigid and easily disaggregated. The result of phase-contrast microscopic observation was as shown in FIG.1. The cell was regularly rounded with a thickness of about 15 micrometers. The Giemsa's stain revealed that the nuclei was rounded occasionally with an irregular lobation or a karyolobism.

(3) Chromosome number

Chromosome analysis was carried out with cells in exponential growth. The frequency distribution of chromosome numbers was as shown in Table I. Observation on 150 chromosomes revealed that the chromosome numbers were in a low diploid region and the most frequent distribution was 45 (53 chromosomes). Forty-two cells had a chromosome number of 44.

Table I

| Frequency distribution of chromosome number | | | | | | | | | | | | | |
|---|---|---|---|---|---|---|---|---|---|---|---|---|---|
| Chromosome number | 31 | 32 | 36 | 40 | 41 | 42 | 43 | 44 | 45 | 46 | 47 | 48 | 49 | 50 |
| Cell number | 1 | 1 | 1 | 1 | 7 | 6 | 11 | 42 | 53 | 14 | 7 | 5 | 1 | 0 |

(4) Karyotypic analysis

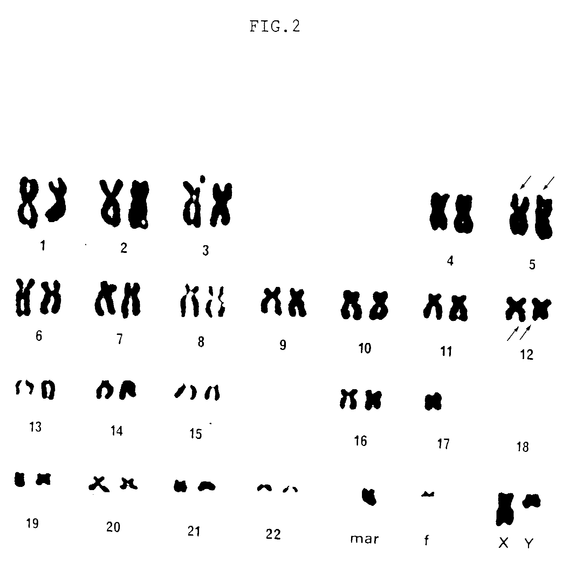

The results of karyotypic analysis were as shown in FIG.2. The sex chromosome was XY, and this was consistent with that of the cell source. One counterpart of chromosome 17 and the whole of chromosome 18 were lacked. It was found that a chromosome was inserted respectively in a short arm (p) of chromosome 5 and a long arm (q) of chromosome 12. An unidentifiable marker chromosome and a chromatid were observed.

(5) Cell surface character

Identification of HBL-38 cell was carried out with various cell surface antibodies, and the results were as shown in Table II. Analysis using goat erythrocyte (E), erythrocyte amboceptor (EA) and erythrocyte amboceptor complement (EAC) revealed that EA formed 10% rosette but the others did not. Detection of surface immunoglobulins (SmIg) using six anti-human goat antibodies revealed that HBL-38 cell was negative to all the antibodies. Screening of surface markers using monoclonal antibodies revealed that 3A1, MCS-2, B3/25 and MY-9 exerted a relatively high positiveness but NU-T2, Leu-5, Leu-4, A-50, BA-2, OKI-1, NU-N1, B2, MO-1 and MO-2 were negative.

(6) Screening of EB virus determined nuclear antigen (EBNA)

HBL-38 cell was screened for EBNA several times from an early stage of the establishment. The results revealed that HBL-38 cell was EBNA-negative.

(7) Colony formation on soft agar medium

HBL-38 cell was tested for colony formation in 0.3% agar medium containing colony stimulating factor (CSF). Inverted-microscopic observation on the 14th day revealed the presence of a colony-forming myeloid cell. The frequency was 1 to 2%. No colony was formed when no CSF was added.

Based on these data, HBL-38 cell was grouped into myelomonocyte.

## Table II Marker profile

### Surface markers

(Standard)

| E | EA | EAC | SmIg | | | | | |
|---|----|-----|------|---|---|---|---|---|
| | | | $\kappa$ | $\lambda$ | $\alpha$ | $\delta$ | $\gamma$ | $\mu$ |
| 0 | 10 | 0 | 0 | 0 | 0 | 0 | 0 | 0 |

(Monoclonal antibody)

| NU-T2 | Leu-5 | Leu-4 | Leu-3a | A50 | 3A1 | Leu-2a | BA2 | NU-N1 | Tac | OKM-1 | MCS-1 |
|-------|-------|-------|--------|-----|-----|--------|-----|-------|-----|-------|-------|
| 0 | 0 | 0 | 10 | 0 | 90 | 10 | 0 | 0 | 10 | 10 | 20 |

| MCS-2 | MY-1 | MY-9 | MO-1 | CA-2-38 | MO-2 | B7/21 | OKI-1 | B3/25 | A3/10 | B2 |
|-------|------|------|------|---------|------|-------|-------|-------|-------|-----|
| 100 | 10 | 70 | 0 | 40 | 0 | 10 | 60 | 100 | 60 | 0 |

### Virus marker

EBV

–

Note: The values indicate positiveness (%).

EP 0 254 593 B1

The following Experiments 1 are illustrative of preparing HuIFN-gamma.

## Experiment 1

Comparison of established human lymphoblastoid cells on HuIFN-gamma producibility

### Experiment 1-1

HuIFN production by in vitro proliferated cell

An established human lymphoblastoid cell was inoculated to RPMI 1640 medium (pH 7.2) supplemented with 20% fetal calf serum, and the mixture was cultured at 37°C in usual manner. The resultant culture was washed with serum-free RPMI 1640 medium (pH 7.2), and then suspended with a fresh preparation of the same culture medium to $1 \times 10^6$ cells/ml.

The resultant cell suspension was added with about 10 μg/ml of lipopolysaccharide, and the mixture was kept at 37°C for two days to induce HuIFN production. The culture was centrifugally separated, and the supernatant was determined for total HuIFN and HuIFN-gamma activities.

The results were as shown in Table III.

As evident from these data, we found that human myelomonocytes was HuIFN-gamma producible and the production was higher than those attained with the established human lymphoblastoid cells tested. HBL-38 cells was extremely high in HuIFN-gamma producibility.

Table III

| T cell | | | |
|---|---|---|---|
| CCRF-CEM | TALL-1 | MOLT-3 | KE-37 |
| below 100 | below 100 | below 100 | below 100 |
| B cell | | | |
| NALM-1 | KLM-2 | Namalwa | BALL-1 |
| below 20 | below 20 | below 20 | below 20 |
| Non T·non B cell | | | |
| KM-3 | NALL-1 | KOPN-K | BV-173 |
| below 20 | below 20 | below 20 | below 20 |
| Non L·non M cell | | | |
| K-562 | HEL | SPI-801 | L-428 |
| below 20 | below 20 | below 20 | below 20 |
| Myelomonocyte | | | |
| HBL-38 | | KG-1 | |
| 3,800 (3,200) | | 1,600 (1,400) | |
| Note: The values indicate HuIFN activity; and those in the parentheses, HuIFN-gamma activity. | | | |

### Experiment 1-2

HuIFN production by in vivo proliferated cell

Newborn hamsters were injected with an antiserum prepared from rabbit in usual manner to weaken possible immuno-reaction, implanted subcutaneously with an established human myelomonocyte, and fed for three weeks in usual manner. The tumor masses formed in the body of the hamsters were extracted and disaggregated by suspension in saline containing trypsin.

A suspension of the obtained cell was treated and determined for total HuIFN and HuIFN-gamma activities similarly as in Experiment 1-1.

The results were as shown in Table IV.

Table IV

| HuIFN production by myelomonocyte | |
| --- | --- |
| HBL-38 | KG-1 |
| 66,000 (59,000) | 31,000 (25,000) |
| Note: The values indicate HuIFN activity; and those in the parentheses, HuIFN-gamma activity. | |

The data in Tables III and IV confirmed that established human myelomonocytes, specifically, HBL-38 cell, exerted a higher HuIFN-gamma producibility when proliferated in vivo than in vitro.

The following Examples are illustrative of preparing HuIFN-gamma.

Example A-1

HBL-38 cell was inoculated on RPMI 1640 medium (pH 7.2), supplemented with 10 v/v % fetal calf serum, to $5 \times 10^5$ cells/ml.

The resultant mixture was cultured at 37°C while periodically refreshing the culture medium. Thereafter, by using a fresh preparation of the same culture medium, the cell was washed and suspended to $2 \times 10^6$ cells/ml. The cell suspension was added with about 10 µg/ml of lipopolysaccharide, and the mixture was kept at 37°C for two days to induce HuIFN production. The resultant culture was centrifugally separated to obtain a supernatant containing about 5,100 units of HuIFN-gamma per ml.

Example A-2

Newborn hamsters were injected with an antiserum prepared from rabbit in usual manner to weaken possible immuno-reaction, implanted subcutaneously with HBL-38 cell, and fed for four weeks in usual manner. The tumor masses, formed in the animals, about 20 g each, were extracted and disaggregated by suspension in saline containing collagenase.

After washing with Eagle's minimal essential medium, the cell was diluted with a fresh preparation of the same culture medium to about $2 \times 10^6$ cells/ml, and added with 200 µg/ml of phytohemagglutinin together with 5 g/ml of lipid A. The mixture was kept at 37°C for two days to induce HuIFN production. The resultant culture was centrifugally separated to obtain a supernatant containing about 93,000 units of HuIFN-gamma. Thus, about 183,000,000 units of HuIFN-gamma was obtained per hamster.

Example A-3

Newborn rats were intravenously implanted with KG-1 cell and fed for four weeks in usual manner.

The tumor masses, about 20 g each, formed in the animals, were extracted and disaggregated similarly as in Example A-2 to obtain a cell suspension. The cell suspension was added with about 100 hemagglutination titers/ml of Sendai virus and about 5 µg/ml of lipopolysaccharide, and the mixture was incubated at 37°C for two days to induce HuIFN production. The resultant culture was centrifugally separated to obtain a supernatant containing about 49,000 units of HuIFN-gamma per ml. The HuIFN-gamma yield was about 97,000,000 units per rat.

Example A-4

CTV-1 cell was suspended with saline in about 10 ml plastic cylindric diffusion chambers having a membrane filter, pore size of about 0.5 microns, and the diffusion chambers were embedded intraperitoneally in adult rats.

The rats were fed for four weeks in usual manner, and the chambers were removed.

The proliferated cell was treated similarly as in Example A-1 to induce HuIFN production. The resultant culture was centrifugally separated to obtain a supernatant containing about 41,000 units of HuIFN-gamma per ml. The yield was about 78,000,000 units per rat.

Example A-5

Embryonated eggs prewarmed at 37°C for one week were implanted with HBL-38 cell, and then incubated at this temperature for an additional one week. The proliferated cells were recovered by breaking the eggs and treated similarly as in Example A-2 to induce HuIFN production. The resultant culture was centrifugally separated to obtain a supernatant containing about 36,000 units of HuIFN-gamma per ml. The yield was about 60,000,000 units per 10 eggs.

The following Examples B are illustrative of preparing monoclonal anti-HuIFN-gamma antibody and of purifying HuIFN-gamma using the same.

Example B-1(1)

Preparation of partially-purified HuIFN-gamma

A liquid containing HuIFN-gamma prepared by the method in Example A-2 was dialyzed against 0.01 M Trisphosphate buffer (pH 8.5) for 20 hours, and then membrane-filtered. The filtrate was applied to an antibody column binding anti-HuIFN-alpha- and anti-HuIFN-beta-antibodies, and the non-adsorbed fraction was recovered. The fraction was chromatofocused to obtain a fraction with an antiviral activity which was then concentrated and lyophilized to obtain a powder containing HuIFN-gamma in the activity of about 30%. The specific activity of the powder was about $10^6$ units/mg protein.

Example B-1(2)

Preparation of monoclonal anti-HuIFN-gamma antibody

A partially-purified HuIFN-gamma obtained by the method in Example B-1(1) was dissolved in saline to about 0.05 w/w %, and the resultant solution was added with an equivalent volume of Freund's complete adjuvant. Mice were injected intravenously with 0.2 ml aliquot of the mixture, and were on the 7th day after the first injection boosted similarly as above to effect immunization. The spleens wherein anti-HuIFN-gamma production had been induced in the antibody producing cell were extracted from the mice, minced and disaggregated, after which the spleen cell was suspended together with $P_3$-X63-Ag8 cell, a mouse myeloma cell purchased from Flow Laboratories Inc., Maryland, USA, in 37°C serum-free Eagle's minimum essential medium (pH 7.2) containing 50 w/v % polyethylene glycol 1000 to give respective cell density of $10^4$ cells/ml. Upon 5-minute standing, the cell suspension was diluted 20 times in a fresh preparation of the same culture medium, and the hybrid cells capable of growing on the hypoxanthine, aminopterin, thymidine containing medium were recovered and cloned in accordance with the method as reported in R.L. Davidson and P.S. Gerald, Somatic Cell Genetics, Vol.2, No.2, pp.175-176 (1976) to obtain a hybrid cell capable of producing anti-HuIFN-gamma antibody. The hybrid cell was then implanted intraperitoneally in mice in a dose of about $10^6$ cell per mouse, fed for two weeks and sacrificed. The body fluids recovered from the mice such as ascite and blood were centrifugally separated to obtain a supernatant which was added with ammonium sulfate to 30-50% saturation. The resultant sediment was dialyzed and affinity-chromatographed with an immobilized HuIFN-gamma gel obtained by reacting at room temperature an HuIFN-gamma that had been obtained by the method in Example B-1(1) with BrCN-activated Sepharose. The obtained anti-HuIFN-gamma antibody fraction was dialyzed, concentrated and lyophilized into powder.

The product immunologically neutralized an HuIFN-gamma derived from human myelomonocyte.

The stability of the monoclonal antibody in aqueous solution was determined by measuring the residual neutralizing activity upon 30-minute incubation at pH 7.2. As a result, the monoclonal antibody retained over 80% activity when incubated at 60°C, but lost over 90% activity when incubated at 70°C. Upon 16-hour incubation at 4°C, the monoclonal antibody was found stable at a pH in the range of 4.0-11.0 but lost over 90% activity at pH 2.0.

Further investigation revealed that the monoclonal antibody was unstable in the presence of 2-mercaptoethanol and caused an antigen-antibody reaction specifically with anti-mouse immunoglobulin M antibody.

This confirmed that the monoclonal antibody was of class immunoglobulin M antibody.

Example B-1(3)

Preparation of highly-purified HuIFN-gamma

A partially-purified HuIFN-gamma prepared by the method in Example B-1(1) was chromatographed on a column of an immobilized monoclonal antibody gel prepared by the method in Example B-1(2), and the fraction with HuIFN-gamma activity was recovered, dialyzed, concentrated and lyophilized to obtain a solid containing HuIFN-gamma in the yield of about 80%. The product was a highly-purified HuIFN-gamma, and the specific activity was about $1.5 \times 10^7$ units/mg protein.

### Example B-2

### Example B-2(1)

#### Preparation of partially-purified HuIFN-gamma

A solution containing an HuIFN-gamma prepared by the method in Example A-3 was partially purified in accordance with the method in Example B-1(1) to obtain an HuIFN-gamma preparation with a specific activity of about $10^6$ units/mg protein in the yield of about 20%.

### Example B-2(2)

#### Preparation of monoclonal anti-HuIFN-gamma antibody

Spleen cell was obtained by immunizing mice similarly as in Example B-1(2), except that the partially-purified HuIFN gamma obtained in Example B-2(1) was used as the antigen.

The spleen cell was suspended together with P3-NS-1/1-Ag4-1 cell, a mouse myeloma cell purchased from Dainippon Pharmaceutical Co., Ltd., Osaka, Japan, in a salt solution containing 140 mM NaCl, 54 mM KCl 1 mM $NaH_2PO_4$ and 2 mM $CaCl_2$ to respective cell density of $10^4$ cells/ml. To the cell suspension was added under ice-chilled conditions a fresh preparation of the same salt solution additionally containing an uv-irradiation inactivated Sendai virus, and the mixture was diluted about 20 times after a lapse of five minutes in 37°C RPMI 1640 medium. A hybrid cell capable of producing anti-HuIFN-gamma antibody was cloned by treating the diluted mixture similarly as in Example B-1(2).

The obtained hybrid cell was implanted intraperitoneally in 7-day old hamsters whose immunoreaction had been weakened in usual manner in a dose of about $10^7$ cells per hamster, and the hamsters were treated similarly as in Example B-1(2) to obtain a monoclonal antibody.

The product immunologically neutralized an HuIFN-gamma derived similarly as the one prepared in Example B-1(2).

The stability of the monoclonal antibody in aqueous solution was determined by measuring the residual neutralizing activity upon 30 minute incubation at pH 7.2. As the result, the monoclonal antibody retained over 80% activity when incubated at 60°C, but lost over 90% activity when incubated at 70°C. Upon 16-hour incubation at 4°C, the monoclonal antibody was found stable at a pH in the range of 2.0-11.0.

Further investigation revealed that the monoclonal antibody was stable in the presence of 2-mercaptoethanol and caused an antigen-antibody reaction specifically with anti-mouse immunoglobulin G antibody.

This confirmed that the monoclonal antibody was of class immunoglobulin G antibody.

### Example B-2(3)

#### Preparation of highly-purified HuIFN-gamma

A partially-purified HuIFN-gamma prepared by the method in Example B-2(1) was chromatographed on a column of an immobilized monoclonal antibody prepared by the method in Example B-2(2), and the fraction containing HuIFN-gamma was recovered, dialyzed and concentrated to obtain a liquid containing HuIFN-gamma in the activity yield of about 85%. The product was a highly-purified HuIFN-gamma, and the specific activity was about $1.5 \times 10^7$ units/mg protein.

### Example B-3

A filtrate obtained by dialyzing an HuIFN-gamma containing supernatant, obtained by the method of Example A-1 was dialyzed against saline containing 0.01 M phosphate buffer (pH 7.2) for 15 hours, and membrane-filtering the resultant supernatant, was purified on an antibody column in accordance with the method in Example B-1(3), concentrated and lyophilized to obtain a solid containing HuIFN-gamma in the activity yield of about 75%. The product was a highly-purified HuIFN-gamma, and the specific activity was about $1.5 \times 10^7$ units/mg protein.

### Example B-4

An HuIFN-gamma containing supernatant obtained by the method in Example A-4 was dialyzed and membrane-filtered in accordance with the method in Example B-3. The resultant filtrate was purified on an antibody column and concentrated similarly as in Example B-2(3) to obtain a solution containing HuIFN-gamma in the activity yield of about 70%. The product was a highly-purified HuIFN-gamma, and the specific activity was about $1.5 \times 10^7$ units/mg protein.

Example B-5

An HuIFN-gamma containing supernatant obtained by the method in Example A-5 was dialyzed and membrane-filtered in accordance with the method in Example B-3. The resultant filtrate was purified on an antibody column, concentrated and lyophilized similarly as in Example B-1(3) to obtain a solid containing HuIFN-gamma in the activity yield of about 70%. The product was a highly-purified HuIFN-gamma, and the specific activity was about $1.5 \times 10^7$ units/mg protein.

The following Experiments 2 are illustrative of preventing and treating HuIFN-gamma susceptive diseases using HuIFN-gamma.

Experiment

Prophylactic and therapeutic effects of HuIFN-gamma on HuIFN-gamma susceptive diseases

Experiment 2-1

Virus inhibitory activity in vitro

A primary monolayer culture of human fetal lung in 6 cm Petri dish was added with an HuIFN-gamma prepared by the method in Example B-1(3) in an inoculum of 0.1, 1.0 or 10.0 units, and incubated in 5% $CO_2$ incubator at 37°C for 20 hours. The resultant culture was added with either varicella-zoster virus or human cytomegalovirus in a dose that was capable of forming 100 plaques in the absence of HuIFN-gamma.

The virus inhibitory activity was determined with the decreasing rate of plaque number.

$$\text{Plaque reduction rate (\%)} = \frac{A - B}{A} \times 100$$

wherein A designates the plaque number with no addition of HuIFN-gamma; B, that with addition of HuIFN-gamma.

The results were as shown in Table V.

Table V

| HuIFN-gamma (units) | Varicella-zoster virus | Cytomegalovirus |
| --- | --- | --- |
| 0.1 | 11% | 10% |
| 1.0 | 54% | 49% |
| 10.0 | 93% | 88% |

These results confirmed that the HuIFN-gamma used in the invention was extremely inhibitory to the growth of pathogenic viruses.

Experiment 2-2

Treatment of malignant tumors with HuIFN-gamma

Experiment 2-2(1)

Cytostatic activity of HuIFN-gamma on malignant tumors in vitro

An aliquot of RPMI 1640 medium supplemented with 15 v/v % fetal calf serum was added with an HuIFN-gamma prepared by the method in Example B-1(3) to a final concentration of 5, 50 or 500 units/ml, and the mixture was inoculated with a malignant human tumor cell to $5 \times 10^5$ cell/ml. The resultant was incubated in 5% $CO_2$ incubator at 37°C for three days. As control, the same amount of an HuIFN-gamma preinactivated by 30-minute incubation at 100°C was added to a fresh preparation of the same culture medium, and the mixture was treated similarly as above. After culture, the viable cell was stained with neutral red in accordance with the method as described in Applied Microbiology, Vol.22, No.4, pp.671-677 (1971). Thereafter, the neutral red was eluted with acidified ethanol, and the number of viable cell was determined with the absorbance of the eluate at 540 nm.

Cytostatic rate (%) was determined with the following equation:

$$\text{Cytostatic rate (\%)} = \left( 1 - \frac{A}{B} \right) \times 100$$

wherein A designates the number of viable cell in the test system, and B, that in the control.

The results were as shown in Table VI.

Table VI

| HuIFN-gamma | Tumor cell | | | |
|---|---|---|---|---|
| (unit/ml) | KB | HEp-2 | KATO-II | P-4788 |
| 5 | 18% | 14% | 28% | 9% |
| 50 | 72% | 31% | 60% | 22% |
| 500 | 93% | 53% | 85% | 43% |
| Note: KB cell is of human oral epidermoid carcinoma origin; HEp-2 cell, human larynx epidermoid carcinoma origin; KATO-II cell, human stomach carcinoma origin; and P-4788, human colon carcinoma origin. | | | | |

These results confirmed that the HuIFN-gamma used in the invention was extremely inhibitory at a concentration of 5-500 units/ml to the growth of malignant tumor cells such as KB cell, HEp-2 cell, KATO-II cell and P-4788 cell.

Experiment 2-2(2)

Potentialization of cytostatic activity of other lymphokines by HuIFN-gamma in vitro

The lymphokines used in this Experiment were HuIFN-gamma (5 units/ml), HuIFN-alpha (50 units/ml) and TNF (10 units/ml). These lymphokines were natural products derived from lymphoblastoid cells.

These lymphokines were tested for cytostatic rate (%) in accordance with the method in Experiment 2-2(1). The results were as shown in Table VII.

Table VII

| Lymphokine | Tumor cell | | | |
|---|---|---|---|---|
| | KB | HEp-2 | KATO-II | P-4788 |
| HuIFN-gamma | 16% | 14% | 26% | 9% |
| HuIFN-alpha | 9% | 6% | 11% | 5% |
| T N F | 5% | 6% | 10% | 6% |
| HuIFN-gamma plus HuIFN-alpha | 45% | 30% | 43% | 27% |
| HuIFN-gamma plus T N F | 70% | 51% | 54% | 42% |
| HuIFN-gamma HuIFN-alpha plus T N F | 92% | 65% | 78% | 61% |

These results evidently confirmed that combination with HuIFN-gamma extremely enhanced the cytostatic effect of other lymphokines on malignant tumors and the enhancement was synergistic.

Experiment 2-2(3)

Potentialization of cytostatic activity of chemotherapeutics on malignant tumors by HuIFN-gamma in vitro

One ml aliquots of a nutrient culture medium prepared in accordance with the method in Experiment 2-2(1) was inoculated with $10^6$ cells of a human malignant tumor cell, and the mixture was cultured for one day. The resultant culture was added with 50 units of an HuIFN-gamma prepared by the method in Example B-1(3) and/or 0.1 ml of saline containing a chemotherapeutic, and cultured at 37°C for two days. As control, a saline free of HuIFN-gamma and chemotherapeutic was used. After the culture, the cytostatic rate (%) was determined in accordance with the method in Experiment 2-2(1). The concentration of the chemotherapeutics were as follows: nimustine hydrochloride (ACNU), $1.0\times10^{-6}$ g/ml; fluorouracil (5-FU), $1.5\times10^{-8}$ g/ml; doxorubicin (ADM), $1.0\times10^{-10}$ g/ml; mitomycin C (MMC), $2.5\times10^{-9}$ g/ml; and vincristine sulfate (VCR), $1.5\times10^{-10}$ g/ml.

The results were as shown in Table VIII.

Table VIII

| Chemotherapeutic | HuIFN-gamma | Tumor cell | | | |
|---|---|---|---|---|---|
| | | HEp-2 | PC-9 | HLE | HeLa |
| - | + | 38% | 53% | 44% | 52% |
| ACNU | - | 4% | 2% | 5% | 5% |
| | + | 54% | 65% | 63% | 72% |
| 5-FU | - | 8% | 10% | 8% | 12% |
| | + | 67% | 68% | 71% | 70% |
| ADM | - | 13% | 6% | 7% | 17% |
| | + | 59% | 72% | 62% | 64% |
| MMC | - | 10% | 5% | 12% | 7% |
| | + | 61% | 64% | 65% | 62% |
| VCR | - | 5% | 8% | 10% | 10% |
| | + | 58% | 66% | 58% | 65% |

Note: (-) means with no addition; and (+) with addition. HEp-2 cell is of human larynx epidermoid carcinoma origin; PC-9, of human lung carcinoma origin; HLE, of human liver carcinoma; and HeLa, of human cervix epitheloid carcinoma origin.

These results evidently confirmed that HuIFN-gamma extremely enhanced the cytostatic activity of chemotherapeutics on malignant tumors and the enhancement was arithmetic or synergistic.

Experiment 2-2(4)

Cytostatic activity on malignant tumors in vivo

BALB/c nude mice were implanted at their dorsal area with a segment of human breast cancer tissue. From the time when the tumors grew to about 200 mm$^3$, the nude mice were injected once every day with one or more HuIFN-gamma prepared by the method in Example B-1(3), a lymphokine derived from human lymphoblastoid cell, and/or a chemotherapeutic in saline solution over a period of 20 days.

Thereafter, the nude mice were sacrificed, and the tumors were weighed.

As control, saline was injected similarly as above. The results were as shown in Table IX.

These results evidently confirmed that HuIFN-gamma extremely inhibited the growth of malignant tumors in vivo. The cytostatic activity was extremely enhanced to exert a strong antioncotic effect by combining one or more lymphokine (s) or chemotherapeutic(s).

Table IX

| Treatment | Dose/kg/day | Tumor (g) |
|---|---|---|
| Control | - | 10.8±+1.0 |
| HuIFN-gamma | 50 units | 6.5+0.9* |
| HuIFN-alpha | 500 units | 7.1+0.8* |
| T N F | 100 units | 6.4±0.5* |
| M M C | 1 mg | 5.1±0.4* |

Table IX   (continued)

| Treatment | Dose/kg/day | Tumor (g) |
|---|---|---|
| HuIFN-gamma plus HuIFN-alpha | 500 units<br>100 units | 4.6±0.5* |
| HuIFN-gamma plus T N F | 50 units<br>100 units | 4.3±0.5* |
| HuIFN-gamma plus M M C | 50 units<br>1 mg | 3.9±0.4* |
| HuIFN-gamma HuIFN-alpha plus T N F | 50 units<br>500 units<br>100 units | 2.7±0.5* |
| HuIFN-gamma HuIFN-alpha plus M M C | 50 units<br>500 units<br>1 mg | 2.6±10.6* |
| Note: The values were stochastically significant against the control at a significance level of 5%. | | |

Experiment 2-2(5)

Acute toxicity

The acute toxicity of an HuIFN-gamma prepartion obtained by the method in Example B-1(3) was tested using 20-day old mice.

The result confirmed that the toxicity of the HuIFNgamma was extremely low, i.e. $10^9$ units or higher in terms of $LD_{50}$, when intraperitoneally injected.

The following Examples C are illustrative of preparing pharmaceutical composition containing as an effective ingredient the HuIFN-gamma prepared by the method according to the invention.

Example C-1

Liquid medicine

A liquid medicine was prepared by dissolving in saline an HuIFN-gamma obtained by the method in Example B-1(3) to 500 units/ml.

The product in the form of nebula, collyrium, collunarium or collutorium is suitable for prophylactic and therapeutic agent for viral diseases such as epidemic conjunctivitis and influenza.

Example C-2

Injection

A sold injection was obtained by dissolving in saline 100,000 units/ml of an HuIFN-gamma prepared by the method in Example B-2(3), sterilely filtering, distributing and lyophilizing 2 ml aliquots of the filtrate in vials, and sealing the vials.

The product is advantageously usable for prophylactic and therapeutic agent for viral diseases similarly as the product in Example C-1.

The product is suitable for prophylactic and therapeutic agent for malignant tumors such as breast cancer, lung carcinoma, liver carcinoma and leukemia; and immunopathies such as atopic allergy, pernicious anemia, articular rheumatism, and systemic lupus erythematosus.

The product is suitable as an enhancer for chemotherapeutics such as melphalan, methotrexate and ADM.

Example C-3

Injection

To saline were added 10,000 units/ml of an HuIFN-gamma prepared by the method in Example B-3, 100,000 units/ ml of natural lymphoblastoid HuIFN-alpha, and 100,000 units/ml of natural lymphoblastoid TNF, and the mixture was sterilely filtered and lyophilized similarly as in Example C-2 to obtain a solid injection.

The product is suitable for prophylactic and therapeutic agent for viral diseases.

The product is also suitable for prophylactic and therapeutic agent for malignant tumors such as breast cancer, lung carcinoma, liver carcinoma, stomach cancer and leukemia; and immunopathies such as atopic allergy, collagenasis, articular rheumatism, and systemic lupus erythematosus.

The product can be advantageously used to enhance the cytostatic activity of chemotherapeutics such as tegafur, MMC and VCR.

Example C-4

Ointment

An HuIFN-gamma prepared by the method in Example B-1(3), and a natural lymphoblastoid HuIFN-alpha were kneaded together with a small amount of liquid paraffin in usual manner, and the mixture was added with white petrolatum to give HuIFN-gamma and HuIFN-alpha concentrations of 50,000 units/g and 500,000 units/g respectively.

The product is suitable for prophylactic and therapeutic agent for skin diseases such as herpes, skin carcinoma, and atopic dermatitis.

Example C-5

Enteric coated tablet

In the preparation of conventional tablet using starch and maltose as vehicle, an HuIFN-gamma prepared by the method in Example B-5 and a natural lymphoblastoid TNF were incorporated into the tablet in respective amount of 10,000 units per tablet (200 mg). The obtained tablets were then coated with methylcellulose phthalate.

The product is advantageously usable for prophylactic and therapeutic agent for viral disease such as those in small and large intestines, as well as that for malignant tumors such as colon carcinoma and liver carcinoma, and immunopathies such as atopic allergy, pernicious anemia, articular rheumatism, and systemic lupus erythematosus.

The product can be advantageously used to enhance the antioncotic activity of chemotherapeutics such as ADM, 5-FU and MMC.

As detailed heretofore, conventional process provides an insufficient HuIFN-gamma productivity and this renders industrial-scale production of HuIFN-gamma very difficult. While based on the finding that established human myelomonocytes exert a superiorly high HuIFN-gamma producibility the present invention provides a process to prepare HuIFN-gamma which is favorably practiceable on an industrial-scale.

The present invention also provides a process to prepare a monoclonal antibody using the HuIFN-gamma thus obtained, as well as providing a purification using the antibody and a prophylactic and therapeutic agent for HuIFN-gamma susceptive diseases. The agent is very effective to viral diseases, malignant tumors and immunopathies, treatment of which has deemed very difficult.

The present invention is a significant invention in the art.

**Claims**

1. A process for preparing interferon-gamma, comprising:

   allowing an established human myelomonocyte capable of producing interferon-gamma to produce interferon-gamma; and
   and recovering the accumulation.

2. A process according to claim 1, wherein said established human myelomonocyte is obtained by:

   implanting an established human myelomonocyte in a non-human warm-blooded animal, or inoculating the established human myelomonocyte in a diffusion chamber placed inside or outside the body of a non-human warm-blooded animal; and

allowing the established human myelomonocyte to proliferate while allowing it to receive the body fluid from the non-human warm-blooded animal.

3. A process according to claim 1 or claim 2, wherein the step of allowing said established human myelomonocyte to produce interferon-gamma includes a step of exposing the established human myelomonocyte to the action of an inducer.

4. A process according to any one of claims 1 to 3, wherein said established human myelomonocyte is a member selected from the group consisting of HL-60 cell, KG-1 cell, ML-1 cell, ML-2 cell, ML-3 cell, THP-1 cell, U-937 cell and CTV-1 cell.

5. A process for preparing monoclonal anti-interferon-gamma antibody, comprising:

allowing an established human myelomonocyte capable of producing interferon-gamma to produce interferon-gamma;
recovering the accumulation;
immunizing a non-human warm-blooded animal using the antigen the obtained interferon-gamma;
recovering the antibody producing cell from the animal;
fusing said antibody producing cell with a myeloma cell;
selecting a hybrid cell capable of producing anti-interferon-gamma antibody; and
culturing the hybrid cell to produce a monoclonal antibody specific to interferon-gamma.

6. A process according to claim 5, wherein said established human myelomonocyte is obtained by:

implanting an established human myelomonocyte in a non-human warm-blooded animal, or inoculating the established human myelomonocyte in a diffusion chamber placed inside or outside the body of a non-human warm-blooded animal; and
allowing the established human myelomonocyte to proliferate while allowing it to receive the body fluid from the non-human warm-blooded animal.

7. A process according to claim 5 or claim 6, wherein said monoclonal antibody is of class immunoglobulin G or M.

8. A method for purifying interferon-gamma, comprising:

allowing an established human myelomonocyte capable of producing interferon- gamma to produce interferon-gamma; and
recovering the accumulation on a column chromatography using an anti-interferon-gamma antibody.

9. A method according to claim 8, wherein said established human myelomonocyte is obtained by:

implanting an established human myelomonocyte in a non-human warm-blooded animal, or inoculating the established human myelomonocyte in a diffusion chamber placed inside or outside the body of a non-human warm-blooded animal; and
allowing the established human myelomonocyte to proliferate while allowing it to receive the body fluid from the non-human warm-blooded animal.

10. A method according to claim 8 or claim 9, wherein said anti-interferon-gamma antibody is obtained by:

allowing an established human myelomonocyte capable of producing interferon-gamma to produce interferon-gamma;
recovering the accumulation;
immunizing a non-human warm-blooded animal using as the antigen the obtained interferon-gamma;
recovering the antibody producing cell from the animal;
fusing said antibody producing cell with a myeloma cell;
selecting a hybrid cell capable of producing anti-interferon-gamma antibody; and
culturing the hybrid cell to produce a monoclonal antibody specific to interferon-gamma.

11. A prophylactic and therapeutic agent for interferon-gamma susceptive diseases, said agent containing as an effective ingredient 1-10,000,000 units of the interferon-gamma per gram of the agent, which interferon-gamma is obtainable by a process according to any one of claims 1 to 4, wherein the interferon-gamma activity is determined by the plaque reduction method using FL cells derived from human amnion, and a unit of the interferon-gamma is defined as the amount that gives a plaque reduction rate (%) of about 50% when calculated by the equation below:

$$\text{Plaque reduction rate (\%)} = \frac{A - B}{A} \times 100$$

wherein "A" designates the plaque number with no addition of the interferon-gamma; and "B", that with addition of the interferon-gamma.

12. An agent according to claim 11, wherein said interferon-gamma has been purified by column chromatography using an anti-interferon-gamma antibody to give a specific activity of about $1.5 \times 10^7$ units/mg of protein.

13. An agent according to claim 11 or claim 12, wherein said agent contains an additional lymphokine.

14. An agent according to claim 13, wherein said lymphokine is a member selected from the group consisting of interferon-alpha, interferon-beta, tumor necrosis factor, lymphotoxin, interleukin 2, B cell differentiating fact, and mixtures thereof.

15. An agent according to any one of claims 11 to 14, wherein said agent acts as an antiviral agent, as an antioncotic agent, as an enhancer for an antioncotic agent, or as a therapeutic agent for immunopathy.

## Patentansprüche

1. Verfahren zur Herstellung von gamma-Interferon, bei dem man einen etablierten menschlichen Myelomonocyt, der gamma-Interferon produzieren kann, gamma-Interferon produzieren läßt und die Ansammlung gewinnt.

2. Verfahren nach Anspruch 1, worin der etablierte menschliche Myelomonocyt erhalten wird, indem man einen etablierten menschlichen Myelomonocyt in ein nicht menschliches Warmbluttier implantiert oder den etablierten menschlichen Myelomonocyt in eine Diffusionskammer, die innerhalb oder außerhalb des Körpers eines nicht menschlichen Warmbluttiers angeordnet ist, impft und den etablierten menschlichen Myelomonocyt proliferieren läßt, während diesem ermöglicht wird, die Körperflüssigkeit von dem nicht menschlichen Warmbluttier zu erhalten.

3. Verfahren nach Anspruch 1 oder Anspruch 2, worin die Stufe, bei der man den etablierten Myelomonocyt gamma-Interferon produzieren läßt, eine Stufe umfaßt, bei der der etablierte menschliche Myelomonocyt der Wirkung eines Induktionsmittels ausgesetzt wird.

4. Verfahren nach einem der Ansprüche 1 bis 3, worin der nachweislich etablierte Myelomonocyt ein Mitglied aus der Gruppe aus HL-60-Zelle, KG-1-Zelle, ML-1-Zelle, ML-2-Zelle, ML-3-Zelle, THP-1-Zelle, U-937-Zelle und CTV-1-Zelle gewählt ist.

5. Verfahren zur Herstellung eines monoklonalen anti-gamma-Interferon-Antikörpers, wobei man

einen etablierten menschlichen Myelomonocyt, der gamma-Interferon produzieren kann, gamma-Interferon produzieren läßt;
die Ansammlung gewinnt;
ein nicht menschliches Warmbluttier immunisiert, wobei als Antigen das erhaltene gamma-Interferon verwendet wird;
die Antikörper-produzierende Zelle aus dem Tier gewinnt; die Antikörper-produzierende Zelle mit einer Myelomzelle verschmilzt;
eine Hybridzelle, die anti-gamma-Interferon-Antikörper produzieren kann, auswählt und
die Hybridzelle züchtet, um einen monoklonalen Antikörper, der spezifisch gegenüber gamma-interferon ist, zu produzieren.

6. Verfahren nach Anspruch 5, worin der etablierte menschliche Myelomonocyt erhalten wird, indem man

einen etablierten menschlichen Myelomonocyt in ein nicht menschliches Warmbluttier implantiert oder den etablierten menschlichen Myelomonocyt in eine Diffusionskammer, die sich innerhalb oder außerhalb des Körpers eines nicht menschlichen Warmbluttiers befindet, impft und

den etablierten menschlichen Myelomonocyt proliferieren läßt, während es ihm ermöglicht wird, die Körperflüssigkeit von dem nicht menschlichen warmbluttier zu erhalten.

7. Verfahren nach Anspruch 5 oder 6, worin der monoklonale Antikörper aus der Klasse Immunglobulin G oder M stammt.

8. Verfahren zum Reinigen von gamma-Interferon, bei dem man

einen etablierten menschlichen Myelomonocyt, der gamma-Interferon produzieren kann, gamma-Interferon produzieren läßt, und

die Ansammlung mittels Säulenchromatographie unter Verwendung eines anti-gamma-Interferon-Antikörpers gewinnt.

9. Verfahren nach Anspruch 8, worin der etablierte menschliche Myelomonocyt erhalten wird, indem man

einen etablierten menschlichen Myelomonocyt in ein nicht menschliches Warmbluttier implantiert oder den etablierten menschlichen Myelomonocyt in eine Diffusionskammer, die sich im innerhalb oder außerhalb des Körpers eines nicht menschlichen Warmbluttiers befindet, impft und

den etablierten menschlichen Myelomonocyt proliferieren läßt, während es ihm ermöglicht wird, die Körperflüssigkeit von dem nicht menschlichen Warmbluttier zu erhalten.

10. Verfahren nach Anspruch 8 oder Anspruch 9, worin der anti-gamma-Interferon-Antikörper erhalten wird, indem man einen etablierten menschlichen Myelomonocyt, der gamma-Interferon produzieren kann, gamma-Interferon produzieren läßt;

die Ansammlung gewinnt;

ein nicht menschliches Warmbluttier unter Verwendung des erhaltenen gamma-Interferons als Antigen immunisiert;

die Antikörper-produzierende Zelle aus dem Tier gewinnt;

die Antikörper-produzierende Zelle mit einer Myelomzelle verschmilzt;

eine Hybridzelle, die anti-gamma-Interferon-Antikörper produzieren kann, auswählt und

die Hybridzelle züchtet, um einen monoklonalen Antikörper, der spezifisch gegenüber gamma-Interferon ist, zu produzieren.

11. Prophylaktisches und therapeutisches Mittel für auf gamma-Interferon sprechende Erkrankungen, wobei das Mittel als wirksamen Bestandteil 1-10.000.000 Einheiten gamma-Interferon pro Gramm des Mittels enthält, welches gamma-Interferon nach dem Verfahren nach einem der Ansprüche 1 bis 4 erhältlich ist, worin die gamma-Interferon-Aktivität nach der Plaquereduktionsmethode unter Verwendung von FL-Zellen aus dem menschlichen Amnion bestimmt wird, und eine Einheit des gamma-Interferons als Menge definiert wird, die eine Plaquereduktionsrate (%) von etwa 50 % ergibt, wenn nach folgender Gleichung berechnet wird:

$$\text{Plaquereduktionsrate (\%)} = \frac{A - B}{A} \times 100$$

worin "A" die Plaquezahl ohne Zugabe von gamma-Interferon und "B" diejenige mit Zugabe des gamma-Interferons bedeuten.

12. Mittel nach Anspruch 11, worin das gamma-Interferon über die Säulenchromatographie unter Verwendung eines anti-gamma-Interferon-Antikörpers gereinigt worden ist, wobei eine spezifische Wirkung von etwa $1{,}51 \times 10^7$ Einheiten/mg Protein erhalten wird.

13. Mittel nach Anspruch 11 oder 12, worin das Mittel ein zusätzliches Lymphokin enthält.

14. Mittel nach Anspruch 13, worin das Lymphokin ein Mitglied aus der Gruppe aus alpha-Interferon, beta-Interferon, Tumornekrosisfaktor, Lymphotoxin, Interleukin-2, B-Zellen-differenzierungsfaktor und Mischungen daraus gewählt ist.

15. Mittel nach einem der Ansprüche 11 bis 14, worin das Mittel als Antivirusmittel, als antionkotisches Mittel, als Verstärker für ein antionkotisches Mittel oder als therapeutisches Mittel für Immunerkrankungen wirkt.

**Revendications**

1. Procédé de préparation d'interféron-gamma, consistant à :

   laisser un myélomonocyte humain établi pouvant produire de l'interféron-gamma produire de l'interféron-gamma; et
   récupérer l'accumulation.

2. Procédé suivant la revendication 1, dans lequel le myélomonocyte humain établi est obtenu en :

   implantant un myélomonocyte humain établi chez un animal à sang chaud non humain, ou inoculant le myélomonocyte humain établi dans une chambre de diffusion placée à l'intérieur ou à l'extérieur du corps d'un animal à sang chaud non humain; et
   laissant le myélomonocyte humain établi proliférer tout en le laissant recevoir le fluide corporel de l'animal à sang chaud non humain.

3. Procédé suivant l'une ou l'autre des revendications 1 et 2, dans lequel l'étape qui consiste à laisser le myélomonocyte humain établi produire de l'interféron-gamma comprend une étape d'exposition du myélomonocyte humain établi à l'action d'un inducteur.

4. Procédé suivant l'une quelconque des revendications 1 à 3, dans lequel le myélomonocyte humain établi est un membre du groupe comprenant la cellule HL-60, la cellule KG-1, la cellule ML-1, la cellule ML-2, la cellule ML-3, la cellule THP-1, la cellule U-937 et la cellule CTV-1.

5. Procédé de préparation d'anticorps anti-interféron-gamma monoclonal,consistant à :

   laisser un myélomonocyte humain établi pouvant produire de l'interféron-gamma produire de l'interféron-gamma;
   récupérer l'accumulation;
   immuniser un animal à sang chaud non humain en utilisant comme antigène l'interféron-gamma obtenu;
   récupérer la cellule productrice d'anticorps de l'animal;
   fusionner la cellule productrice d'anticorps avec une cellule de myélome;
   sélectionner une cellule hybride pouvant produire un anticorps anti-interféron-gamma; et
   cultiver la cellule hybride pour produire un anticorps monoclonal spécifique à l'interféron-gamma.

6. Procédé suivant la revendication 5, dans lequel le myélomonocyte humain établi est obtenu en :

   implantant un myélomonocyte humain établi chez un animal à sang chaud non humain, ou inoculant le myélomonocyte humain établi dans une chambre de diffusion placée à l'intérieur ou à l'extérieur du corps d'un animal à sang chaud non humain; et
   laissant le myélomonocyte humain établi proliférer tout en le laissant recevoir le fluide corporel de l'animal à sang chaud non humain.

7. Procédé suivant l'une ou l'autre des revendications 5 et 6, dans lequel l'anticorps monoclonal est de la classe de l'immunoglobuline G ou M.

8. Procédé de purification d'interféron-gamma, consistant à :

   laisser un myélomonocyte humain établi pouvant produire de l'interféron-gamma produire de l'interféron-gamma; et
   récupérer l'accumulation sur une chromatographie sur colonne en utilisant un anticorps anti-interféron-gamma.

9. Procédé suivant la revendication 8, dans lequel le myélomonocyte humain établi est obtenu en :

implantant un myélomonocyte humain établi chez un animal à sang chaud non humain, ou inoculant le myélomonocyte humain établi dans une chambre de diffusion placée à l'intérieur ou à l'extérieur du corps d'un animal à sang chaud non humain; et
laissant le myélomonocyte humain établi proliférer tout en le laissant recevoir le fluide corporel de l'animal à sang chaud non humain.

10. Procédé suivant l'une ou l'autre des revendications 8 et 9, dans lequel l'anticorps anti-interféron-gamma est obtenu en :

laissant un myélomonocyte humain établi pouvant produire de l'interféron-gamma produire de l'interféron-gamma;
récupérant l'accumulation;
immunisant un animal à sang chaud non humain en utilisant comme antigène l'interféron-gamma obtenu;
récupérant la cellule productrice d'anticorps de l'animal;
fusionnant la cellule productrice d'anticorps avec une cellule de myélome;
sélectionnant une cellule hybride pouvant produire un anticorps anti-interféron-gamma; et
cultivant la cellule hybride pour produire un anticorps monoclonal spécifique à l'interféron-gamma.

11. Agent prophylactique et thérapeutique pour maladies sensibles à l'interféron-gamma, ledit agent contenant comme ingrédient efficace 1-10.000.000 unités de l'interféron-gamma par gramme de l'agent, lequel interféron-gamma est obtenable par un procédé suivant l'une quelconque des revendications 1 à 4, dans lequel l'activité d'interféron-gamma est déterminée par la méthode de réduction de plaques en utilisant des cellules FL provenant d'amnios humain, et une unité de l'interféron-gamma est définie par la quantité qui donne un taux de réduction de plaques (%) d'environ 50 % lorsque calculé par l'équation suivante :

$$\textit{Taux de réduction de plaques } (\%) = \frac{A - B}{A} \textit{ x } 100$$

dans laquelle "A" désigne le nombre de plaques sans addition de l'interféron-gamma et "B" celui avec addition de l'interféron-gamma.

12. Agent suivant la revendication 11, dans lequel l'interféron-gamma a été purifié par chromatographie sur colonne en utilisant un anticorps anti-interféron-gamma pour donner une activité spécifique d'environ 1,5 x $10^7$ unités/mg de protéine.

13. Agent suivant l'une ou l'autre des revendications 11 et 12, dans lequel ledit agent contient une lymphokine additionnelle.

14. Agent suivant la revendication 13, dans lequel la lymphokine est un membre choisi dans le groupe comprenant l'interféron-alpha, l'interféron-bêta, le facteur de nécrose tumorale, la lymphotoxine, l'interleukine-2, le facteur de différenciation de cellules B et leurs mélanges.

15. Agent suivant l'une quelconque des revendications 11 à 14, dans lequel ledit agent sert d'agent antiviral, d'agent antioncotique, d'activateur pour agent antioncotique ou d'agent thérapeutique pour immunopathie.

FIG.1

FIG.2